# EUROPEAN PATENT APPLICATION

(11) **EP 4 393 387 A2**
(43) Date of publication of application: **03.07.2024**
(21) Application number: 24168778.9
(22) Date of filing: 08.06.2020
(51) Int. Cl.: A61B 5/053

(54) **POSTURE DETERMINATION AND STIMULATION ADJUSTMENT IN A SPINAL CORD STIMULATOR SYSTEM USING SENSED STIMULATION ARTIFACTS**

(30) Priority: 12.06.2019 US 201962860627 P
(62) Divisional of application: 20750479.6
(71) Applicant: Boston Scientific Neuromodulation Corporation, Valencia, CA 91355 (US)
(72) Inventor: The designation of the inventor has not yet been filed
(74) Representative: Vossius & Partner Patentanwälte Rechtsanwälte mbB

(57) **Abstract**

The present invention relates to a stimulator device, comprising a plurality of electrodes configured to contact a patient's tissue and stimulation circuitry configured to provide stimulation at at least two of the electrodes. The stimulation induces an electric field in the tissue, wherein the electric field is configured to recruit neural fibers in the tissue causing a neural response. The stimulation device further comprises a sense amplifier configured to sense a signal over time at a sensing electrode comprising one of the electrodes different from the at least two electrodes that provide the stimulation. The signal comprises a stimulation artifact formed by the electric field and the neural response. The stimulation device also comprises control circuitry configured to process the sensed signal to determine at least one feature of the stimulation artifact and to determine at least one feature of the neural response.

## Description

### FIELD OF THE INVENTION

This application relates to Implantable Medical Devices (IMDs), and more specifically sensing signals in an implantable stimulator device.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227. However, the present invention may find applicability with any implantable neurostimulator device system.

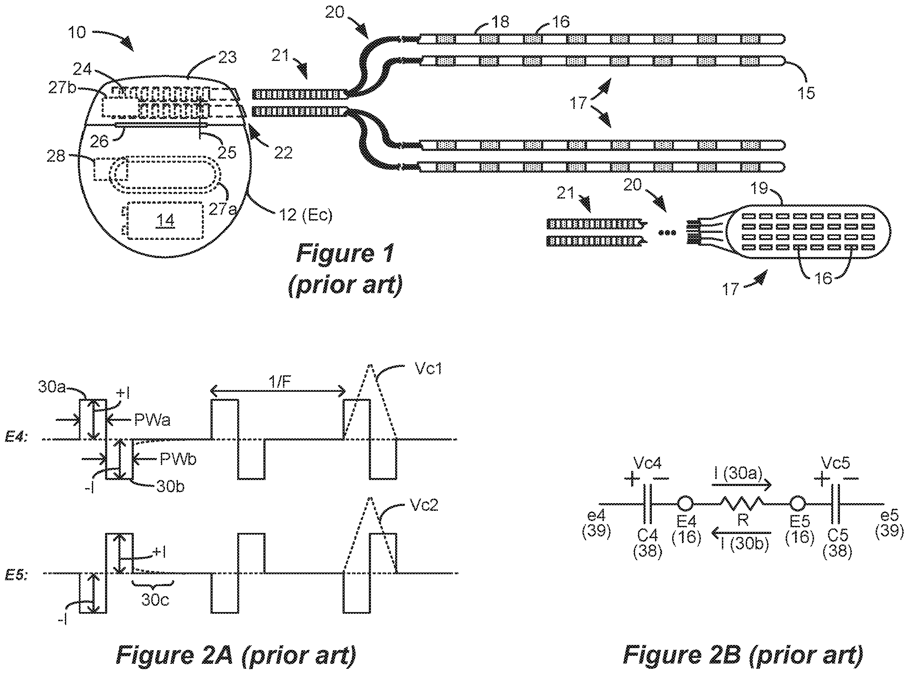

An SCS system typically includes an Implantable Pulse Generator (IPG) 10 shown in Figure 1. The IPG 10 includes a biocompatible device case 12 that holds the circuitry and a battery 14 for providing power for the IPG to function. The IPG 10 is coupled to tissue-stimulating electrodes 16 via one or more electrode leads that form an electrode array 17. For example, one or more percutaneous leads 15 can be used having ring-shaped or split-ring electrodes 16 carried on a flexible body 18. In another example, a paddle lead 19 provides electrodes 16 positioned on one of its generally flat surfaces. Lead wires 20 within the leads are coupled to the electrodes 16 and to proximal contacts 21 insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12.

In the illustrated IPG 10, there are thirty-two electrodes (E1-E32), split between four percutaneous leads 15, or contained on a single paddle lead 19, and thus the header 23 may include a 2x2 array of eight-electrode lead connectors 22. However, the type and number of leads, and the number of electrodes, in an IPG is application specific and therefore can vary. The conductive case 12 can also comprise an electrode (Ec). In a SCS application, the electrode lead(s) are typically implanted in the spinal column proximate to the dura in a patient's spinal cord, preferably spanning left and right of the patient's spinal column. The proximal contacts 21 are tunneled through the patient's tissue to a distant location such as the buttocks where the IPG case 12 is implanted, at which point they are coupled to the lead connectors 22. In other IPG examples designed for implantation directly at a site requiring stimulation, the IPG can be lead-less, having electrodes 16 instead appearing on the body of the IPG 10 for contacting the patient's tissue. The IPG lead(s) can be integrated with and permanently connected to the IPG 10 in other solutions. The goal of SCS therapy is to provide electrical stimulation from the electrodes 16 to alleviate a patient's symptoms, such as chronic back pain.

IPG 10 can include an antenna 27a allowing it to communicate bi-directionally with a number of external devices used to program or monitor the IPG, such as a hand-held patient controller or a clinician's programmer, as described for example in U.S. Patent Application Publication 2019/0175915. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Zigbee, MICS, and the like.

Stimulation in IPG 10 is typically provided by pulses each of which may include a number of phases such as 30a and 30b, as shown in the example of Figure 2A. Stimulation parameters typically include amplitude (current I, although a voltage amplitude V can also be used); frequency (F); pulse width (PW) of the pulses or of its individual phases; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E4 has been selected as an anode (during its first phase 30a), and thus provides pulses which source a positive current of amplitude +I to the tissue. Electrode E5 has been selected as a cathode (again during first phase 30a), and thus provides pulses which sink a corresponding negative current of amplitude -I from the tissue. This is an example of bipolar stimulation, in which only two lead-based electrodes are used to provide stimulation to the tissue (one anode, one cathode). However, more than one electrode may be selected to act as an anode at a given time, and more than one electrode may be selected to act as a cathode at a given time.

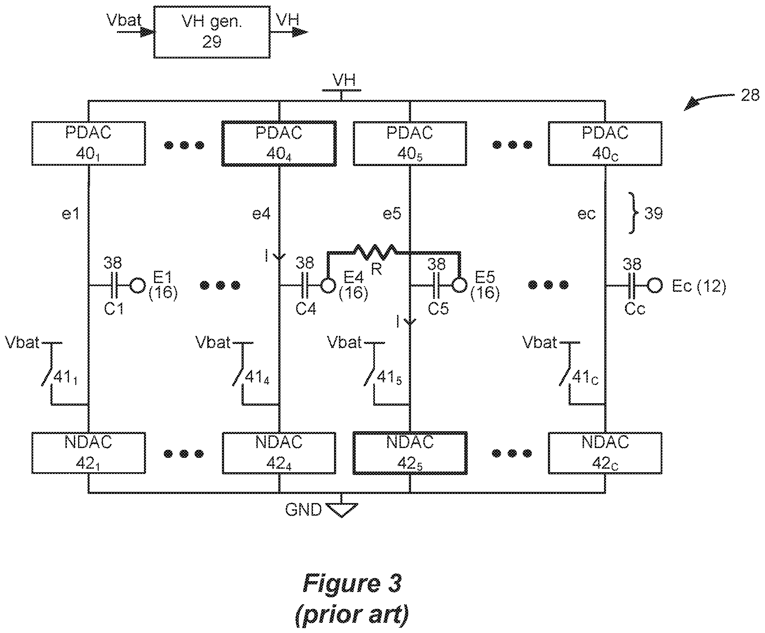

IPG 10 as mentioned includes stimulation circuitry 28 to form prescribed stimulation at a patient's tissue. Figure 3 shows an example of stimulation circuitry 28, which includes one or more current source circuits 40ᵢ and one or more current sink circuits 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node ei 39. Each electrode node ei 39 is connected to an electrode Ei 16 via a DC-blocking capacitor Ci 38, for the reasons explained below. The stimulation circuitry 28 in this example also supports selection of the conductive case 12 as an electrode (Ec 12), which case electrode is typically selected for monopolar stimulation. PDACs 40ᵢ and NDACs 42ᵢ can also comprise voltage sources.

Proper control of the PDACs 40ᵢ and NDACs 42ᵢ allows any of the electrodes 16 to act as anodes or cathodes to create a current through a patient's tissue, R, hopefully with good therapeutic effect. In the example shown (Fig. 2A), and during the first phase 30a in which electrodes E4 and E5 are selected as an anode and cathode respectively, PDAC 40₄ and NDAC 42₅ are activated and digitally programmed to produce the desired current, I, with the correct timing (e.g., in accordance with the prescribed frequency F and pulse widths PWa). During the second phase 30b (PWb), PDAC 40s and NDAC 42₄ would be activated to reverse the polarity of the current. More than one anode electrode and more than one cathode electrode may be selected at one time, and thus current can flow through the tissue R between two or more of the electrodes 16.

Power for the stimulation circuitry 28 is provided by a compliance voltage VH. As described in further detail in U.S. Patent Application Publication 2013/0289665, the compliance voltage VH can be produced by a compliance voltage generator 29, which can comprise a circuit used to boost the battery 14's voltage (Vbat) to a voltage VH sufficient to drive the prescribed current I through the tissue R. The compliance voltage generator 29 may comprise an inductor-based boost converter as described in the '665 Publication, or can comprise a capacitor-based charge pump. Because the resistance of the tissue is variable, VH may also be variable, and can be as high as 18 Volts in one example.

Other stimulation circuitries 28 can also be used in the IPG 10. In an example not shown, a switching matrix can intervene between the one or more PDACs 40; and the electrode nodes ei 39, and between the one or more NDACs 42ᵢ and the electrode nodes. Switching matrices allows one or more of the PDACs or one or more of the NDACs to be connected to one or more anode or cathode electrode nodes at a given time. Various examples of stimulation circuitries can be found in USPs 6,181,969, 8,606,362, 8,620,436, and U.S. Patent Application Publications 2018/0071520 and 2019/0083796. Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, and 2012/0095519. As explained in these references, ASIC(s) may also contain other circuitry useful in the IPG 10, such as telemetry circuitry (for interfacing off chip with telemetry antennas 27a and/or 27b), the compliance voltage generator 29, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Although not shown, circuitry in the IPG 10 including the stimulation circuitry 28 can also be included in an External Trial Stimulator (ETS) device which is used to mimic operation of the IPG during a trial period and prior to the IPG 10's implantation. An ETS device is typically used after the electrode array 17 has been implanted in the patient. The proximal ends of the leads in the electrode array 17 pass through an incision in the patient and are connected to the externally-worn ETS, thus allowing the ETS to provide stimulation to the patient during the trial period. Further details concerning an ETS device are described in USP 9,259,574 and U.S. Patent Application Publication 2019/0175915.

Referring again to Figure 2A, the stimulation pulses as shown are biphasic, with each pulse at each electrode comprising a first phase 30a followed thereafter by a second phase 30b of opposite polarity. Biphasic pulses are useful to actively recover any charge that might be stored on capacitive elements in the electrode current paths, such as the DC-blocking capacitors 38, the electrode/tissue interface, or within the tissue itself. To recover all charge by the end of the second pulse phase 30b of each pulse (Vc4 = Vc5 = 0V), the first and second phases 30a and 30b are preferably charged balanced at each electrode, with the phases comprising an equal amount of charge but of the opposite polarity. In the example shown, such charge balancing is achieved by using the same pulse width (PWa = PWb) and the same amplitude (|+I| = |-I||) for each of the pulse phases 30a and 30b. However, the pulse phases 30a and 30b may also be charged balance if the product of the amplitude and pulse widths of the two phases 30a and 30b are equal, as is known.

Figure 3 shows that stimulation circuitry 28 can include passive recovery switches 41ᵢ, which are described further in U.S. Patent Application Publications 2018/0071527 and 2018/0140831. Passive recovery switches 41ᵢ may be attached to each of the electrode nodes 39, and are used to passively recover any charge remaining on the DC-blocking capacitors Ci 38 after issuance of the second pulse phase 30b-i.e., to recover charge without actively driving a current using the DAC circuitry. Passive charge recovery can be prudent, because non-idealities in the stimulation circuitry 28 may lead to pulse phases 30a and 30b that are not perfectly charge balanced. Passive charge recovery typically occurs during at least a portion 30c (Fig. 2A) of the quiet periods between the pulses by closing passive recovery switches 41ᵢ. As shown in Figure 3, the other end of the switches 41ᵢ not coupled to the electrode nodes 39 are connected to a common reference voltage, which in this example comprises the voltage of the battery 14, Vbat, although another reference voltage could be used. As explained in the above-cited references, passive charge recovery tends to equilibrate the charge on the DC-blocking capacitors 38 and other capacitive elements by placing the capacitors in parallel between the reference voltage (Vbat) and the patient's tissue. Note that passive charge recovery is illustrated as small exponentially-decaying curves during 30c in Figure 2A, which may be positive or negative depending on whether pulse phase 30a or 30b has a predominance of charge at a given electrode.

### SUMMARY

A method is disclosed for operating a stimulator device, the stimulator device comprising a plurality of electrodes configured to contact a patient's tissue. The method may comprises: providing stimulation at at least two of the electrodes; sensing a stimulation artifact over time at a sensing electrode comprising one of the electrodes different from the at least two electrodes that provide the stimulation, wherein the stimulation artifact comprises a signal formed by an electric field induced in the tissue by the stimulation; determining at least one feature of the stimulation artifact; and using at least the determined at least one stimulation artifact feature to adjust the stimulation.

In one example, the electric field is configured to recruit neural fibers in the tissue causing a neural response. In one example, the method may further comprise: sensing the neural response at the sensing electrode; determining at least one feature of the neural response; and using the determined at least one neural response feature to adjust the stimulation. In one example, the sensed stimulation artifact excludes the neural response. In one example, sensing the stimulation artifact comprises subtracting the neural response from the stimulation artifact. In one example, the stimulation artifact occurs before arrival of the neural response at the sensing electrode. In one example, the method may further comprise using the determined at least one stimulation artifact feature to determine a posture or activity of the patient. In one example, the stimulator device is programmed with a database associating values or ranges of values of the at least one stimulation artifact with different of the postures or activities. In one example, determining at least one feature of the stimulation artifact comprises determining a value for the stimulation artifact feature, and wherein using the determined at least one stimulation artifact feature to determine a posture or activity of the patient comprises using the determined stimulation artifact feature value to select from the database one of the postures or activities that is associated with a value or range of values that matches the determined stimulation artifact feature value. In one example, the stimulator device is programmed with a database associating values or ranges of values of the at least one stimulation artifact with different stimulation programs. In one example, determining at least one feature of the stimulation artifact comprises determining a value for the stimulation artifact feature, and wherein using the determined at least one stimulation artifact feature to adjust the stimulation comprises using the determined stimulation artifact feature value to select from the database one the stimulation programs that is associated with a value or range of values that matches the determined stimulation artifact feature value. In one example, the stimulation artifact is sensed at a sense amplifier in the stimulation device. In one example, the stimulation artifact is sensed at the sense amplifier in a single-ended manner using a fixed reference potential as a reference. In one example, the stimulation artifact is sensed at the sense amplifier differentially using another one of the electrodes as a reference. In one example, the stimulation artifact is not blanked at the sense amplifier. In one example, a DC-blocking capacitor intervenes between the sensing electrode at the sense amplifier. In one example, the at least one stimulation artifact feature is indicative of an energy of the stimulation artifact. In one example, the at least one stimulation artifact feature is determined over a time period. In one example, the electric field is configured to recruit neural fibers in the tissue causing a neural response detectable at the sensing electrode, wherein the time period excludes the neural response. In one example, the at least two electrodes are spaced at a distance from the sensing electrode, and further comprising using the distance to adjust the stimulation. In one example, the stimulation has an amplitude, and further comprising using the amplitude to adjust the stimulation. In one example, the method further comprises selecting the sensing electrode from one of the electrodes. In one example, the stimulator device comprises a Spinal Cord Stimulator device. In one example, the method is repeated to continually adjust the stimulation.

A method is disclosed for operating a stimulator device, the stimulator device comprising a plurality of electrodes configured to contact a patient's tissue. The method may comprise: providing stimulation at at least two of the electrodes; sensing a stimulation artifact over time at a sensing electrode comprising one of the electrodes different from the at least two electrodes that provide the stimulation, wherein the stimulation artifact comprises a signal formed by an electric field induced in the tissue by the stimulation; determining at least one feature of the stimulation artifact; and using at least the determined at least one stimulation artifact feature to determine a posture or activity of the patient.

In one example, the electric field is configured to recruit neural fibers in the tissue causing a neural response. In one example, the method may further comprise: sensing the neural response at the sensing electrode; determining at least one feature of the neural response; and using the determined at least one neural response feature to determine the posture or activity of the patient. In one example, the sensed stimulation artifact excludes the neural response. In one example, sensing the stimulation artifact comprises subtracting the neural response from the stimulation artifact. In one example, sensing the stimulation artifact occurs before arrival of the neural response at the sensing electrode. In one example, the stimulator device is programmed with a database associating values or ranges of values of the at least one stimulation artifact with different of the postures or activities. In one example, determining at least one feature of the stimulation artifact comprises determining a value for the stimulation artifact feature, and wherein using the determined at least one stimulation artifact feature to determine a posture or activity of the patient comprises using the determined stimulation artifact feature value to select from the database one of the postures or activities that is associated with a value or range of values that matches the determined stimulation artifact feature value. In one example, the stimulation artifact is sensed at a sense amplifier in the stimulation device. In one example, the stimulation artifact is sensed at the sense amplifier in a single-ended manner using a fixed reference potential as a reference. In one example, the stimulation artifact is sensed at the sense amplifier differentially using another one of the electrodes as a reference. In one example, the stimulation artifact is not blanked at the sense amplifier. In one example, a DC-blocking capacitor intervenes between the sensing electrode at the sense amplifier. In one example, the at least one stimulation artifact feature is indicative of an energy of the stimulation artifact. In one example, the at least one stimulation artifact feature is determined over a time period. In one example, the electric field is configured to recruit neural fibers in the tissue causing a neural response detectable at the sensing electrode, wherein the time period excludes the neural response. In one example, the at least two electrodes are spaced at a distance from the sensing electrode, and further comprising using the distance to determine the posture or activity of the patient. In one example, the stimulation has an amplitude, and further comprising using the amplitude to determine the posture or activity of the patient. In one example, the method further comprises selecting the sensing electrode from one of the electrodes. In one example, the stimulator device comprises a Spinal Cord Stimulator device. In one example, the method further comprises transmitting the determined posture or activity of the patient to an external device. In one example, the method further comprises storing a log of the determined posture or activity of the patient as a function of time in the stimulator device. In one example, the method further comprises transmitting the log to an external device. In one example, the activity of the patient comprises sleep or a resting state.

A method is disclosed for operating a stimulator device, the stimulator device comprising a plurality of electrodes configured to contact a patient's tissue. The method may comprise: providing stimulation at at least two of the electrodes, wherein the stimulation induces an electric field in the tissue, wherein the electric field is configured to recruit neural fibers in the tissue causing a neural response; sensing a signal over time at a sensing electrode comprising one of the electrodes different from the at least two electrodes that provide the stimulation, wherein the signal comprises a stimulation artifact formed by the electric field and the neural response; and processing the sensed signal to determine at least one feature of the stimulation artifact and to determine at least one feature of the neural response.

In one example, the sensed signal is digitized prior to processing the sensed signal. In one example, the signal is sensed at a sense amplifier in the stimulation device. In one example, the signal is sensed at the sense amplifier in a single-ended manner using a fixed reference potential as a reference. In one example, the signal is sensed at the sense amplifier differentially using another one of the electrodes as a reference. In one example, the signal is not blanked at the sense amplifier. In one example, a DC-blocking capacitor intervenes between the sensing electrode at the sense amplifier. In one example, the at least one stimulation artifact feature is indicative of an energy of the stimulation artifact. In one example, the at least one stimulation artifact feature is determined over a time period. In one example, the time period excludes the neural response. In one example, the method further comprises selecting the sensing electrode from one of the electrodes. In one example, the stimulator device comprises a Spinal Cord Stimulator device. In one example, the method further comprises using one or more of the determined at least one stimulation artifact feature and the determined at least one neural response feature to adjust the stimulation. In one example, the method is repeated to continually adjust the stimulation. In one example, the stimulator device is programmed with a database associating different stimulation programs with values or ranges of values of the at least one stimulation artifact feature or with values or ranges of the at least one neural response feature. In one example, the method further comprises using one or more of the determined at least one stimulation artifact feature and the determined at least one neural response feature to determine a posture or activity of the patient. In one example, the stimulator device is programmed with a database associating different of the postures or activities with values or ranges of values of the at least one stimulation artifact or with values or ranges of the at least one neural response feature. In one example, the method further comprises transmitting the determined posture or activity of the patient to an external device. In one example, the method further comprises storing a log of the determined posture or activity of the patient as a function of time in the stimulator device. In one example, the method further comprises transmitting the log to an external device.

### BRIEF DESCRIPTION OF THE DRAWINGS

Figure 1 shows an Implantable Pulse Generator (IPG), in accordance with the prior art.
Figures 2A and 2B show an example of stimulation pulses producible by the IPG, in accordance with the prior art.
Figure 3 shows stimulation circuitry useable in the IPG, in accordance with the prior art.
Figures 4A and 4B show an improved IPG having sensing capability, and the ability to adjust stimulation dependent on such sensing.
Figures 5A-5B show stimulation producing a neural response such as an ECAP, and Figure 5C shows sensing of the stimulation artifact in the ElectroSpinoGram (ESG) signal caused by the stimulation as well and the sensed neural response. Figures 5D and 5E respectively show how the amplitude of stimulation artifacts and ECAPs vary as a function of the distance between sensing and stimulation.
Figure 6 shows a system used during experimentation to determine the relevance of certain stimulation artifact features and certain neural response features to distinguishing changes in patient posture.
Figure 7 shows weights denoting the relevance of the features determined during testing using the system of Figure 6, which shows the relevance of stimulation artifact features in discriminating between different patient posture states.
Figure 8 shows differences in a relevant stimulation artifact feature (total energy from 0-0.4 ms) at different stimulation current amplitudes I and different stimulation-to-sense distances d.
Figures 9A-9C show different examples in which measured stimulation artifact features can be used (perhaps along with other variables) in an IPG to determine patient posture or activity, or to adjust the stimulation program or parameters provided to the patient.
Figure 10 shows a system that can be used to program the IPG during a patient fitting session to enable use of stimulation artifact feature sensing.
Figure 11 shows a system in which data logged in the IPG can be transmitted to an external device for review, including data relevant to patient posture and activity.
Figures 12A-12D show actual ESG signals including stimulation artifacts and ECAPs in mammalian subjects.

### DETAILED DESCRIPTION

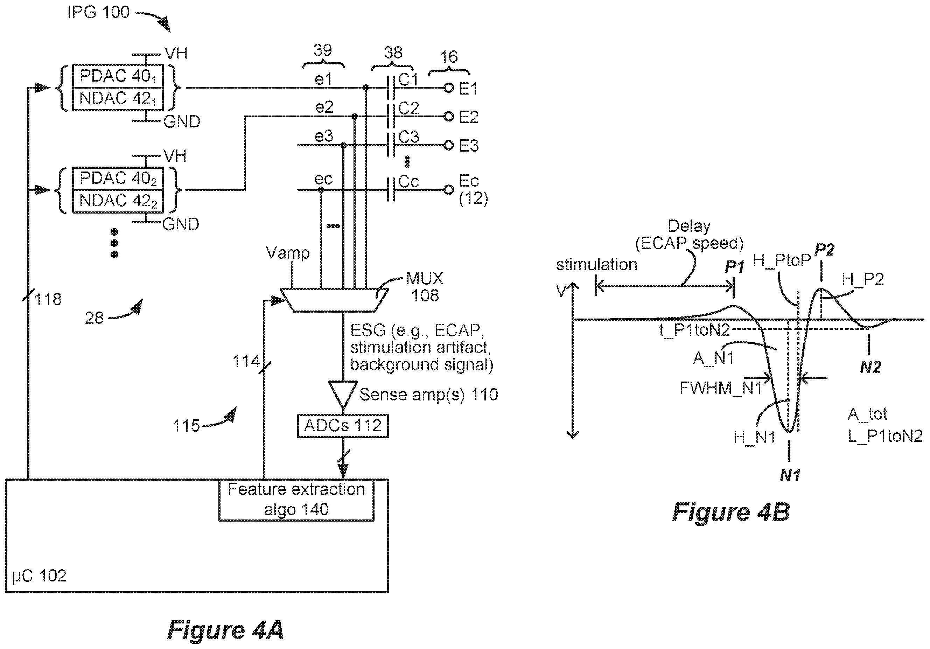

An increasingly interesting development in pulse generator systems, and in Spinal Cord Stimulator (SCS) pulse generator systems specifically, is the addition of sensing capability to complement the stimulation that such systems provide. For example, and as explained in U.S. Patent Application Publication 2017/0296823, it can be beneficial to sense a neural response in neural tissue that has received stimulation from an SCS pulse generator. One such neural response is an Evoked Compound Action Potential (ECAP). An ECAP comprises a cumulative response provided by neural fibers that are recruited by the stimulation, and essentially comprises the sum of the action potentials of recruited neural elements (ganglia or fibers) when they "fire." An ECAP is shown in Figure 4B, and comprises a number of peaks that are conventionally labeled with P for positive peaks and N for negative peaks, with P1 comprising a first positive peak, N1 a first negative peak, P2 a second positive peak and so on. Note that not all ECAPs will have the exact shape and number of peaks as illustrated in Figure 4B, because an ECAP's shape is a function of the number and types of neural elements that are recruited and that are involved in its conduction. An ECAP is generally a small signal, and may have a peak-to-peak amplitude on the order of units to hundreds of microVolts depending on the amplification gain and location within the nervous system where these are sensed (brain, spinal cord, peripheral nervous system, somatic nervous system, motor elements, or other).

Shown in Figure 4A is circuitry for an IPG 100 that is capable of providing stimulation and sensing an ElectroSpinoGram (ESG) signal. (This circuitry could also be present in an ETS as described earlier, although use in an IPG is discussed for simplicity). The ESG signal, as explained further below, can include various pieces of information, such as an ECAP or other neural response to stimulation, a stimulation artifact arising from the stimulation provided to the tissue, and other background signals that may be produced by neural tissue even absent stimulation. The IPG 100 includes control circuitry 102, which may comprise a microcontroller for example such as Part Number MSP430, manufactured by Texas Instruments, which is described in data sheets at http://www.ti.com/ lsds/ ti/ microcontroller/ 16-bit_msp430/ overview.page? DCMP = MCU_other& HQS = msp430. Other types of controller circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), such as those described earlier.

The IPG 100 also includes stimulation circuitry 28 to produce stimulation at the electrodes 16, which may comprise the stimulation circuitry 28 shown earlier (Fig. 3). A bus 118 provides digital control signals from the control circuitry 102 (and possibly from an feature extraction algorithm 140, described below) to one or more PDACs 40ᵢ or NDACs 42ᵢ to produce currents or voltages of prescribed amplitudes (I) for the stimulation pulses, and with the correct timing (PW, F). As noted earlier, the DACs can be powered between a compliance voltage VH and ground. As also noted earlier, but not shown in Figure 4A, switch matrices could intervene between the PDACs and the electrode nodes 39, and between the NDACs and the electrode nodes, to route their outputs to one or more of the electrodes, including the conductive case electrode 12 (Ec). Control signals for switch matrices, if present, may also be carried by bus 118. Notice that the current paths to the electrodes 16 include the DC-blocking capacitors 38 described earlier, which provide safety by preventing the inadvertent supply of DC current to an electrode and to a patient's tissue. Passive recovery switches 41ᵢ (Fig. 3) could also be present, but are not shown in Figure 4A for simplicity.

IPG 100 also includes sensing circuitry 115, and one or more of the electrodes 16 can be used to sense signals the ESG signal. In this regard, each electrode node 39 is further coupleable to a sense amp circuit 110. Under control by bus 114, a multiplexer 108 can select one or more electrodes to operate as sensing electrodes by coupling the electrode(s) to the sense amps circuit 110 at a given time, as explained further below. Although only one multiplexer 108 and sense amp circuit 110 is shown in Figure 4A, there could be more than one. For example, there can be four multiplexer 108/sense amp circuit 110 pairs each operable within one of four timing channels supported by the IPG 100 to provide stimulation. The sensed signal are preferably converted to digital signals by one or more Analog-to-Digital converters (ADC(s)) 112, which may sample the waveform at 50 kHz for example. The ADC(s) 112 may also reside within the control circuitry 102, particularly if the control circuitry 102 has A/D inputs. Multiplexer 108 can also provide a fixed reference voltage, Vamp, to the sense amp circuit 110, as is useful in a single-ended sensing mode.

So as not to bypass the safety provided by the DC-blocking capacitors 38, the input to the sense amp circuitry 110 is preferably taken from the electrode nodes 39, and so the DC-blocking capacitors 38 intervene between the electrodes 16 where the signals are sensed and the electrode nodes 39. However, the DC-blocking capacitors 38 will pass AC signal components while blocking DC components, and thus AC signals will still readily be sensed by the sense amp circuit 110. In other examples, signals may be sensed directly at the electrodes 16 without passage through intervening capacitors 38.

As shown, a feature extraction algorithm 140 is programmed into the control circuitry 102 to receive and analyze the digitized sensed signals. One skilled in the art will understand that the feature extraction algorithm 140 can comprise instructions that can be stored on non-transitory machine-readable media, such as magnetic, optical, or solid-state memories within the IPG 100 (e.g., stored in association with control circuitry 102).

The feature extraction algorithm 140 operates within the IPG 100 to determine one or more features, generally speaking by analyzing the size and shape of the sensed signals. For an ECAP as described earlier, the feature extraction algorithm 140 can determine one or more ECAP features (EFx), which may include but are not limited to:
- a height of any peak (e.g., H_N1) present in the ECAP;
- a peak-to-peak height between any two peaks (such as H_PtoP from N1 to P2);
- a ratio of peak heights (e.g., H_N1 / H_P2);
- a peak width of any peak (e.g., the full width half maximum of a N1, FWHM_N1);
- an area under any peak (e.g., A_N1);
- a total area (A_tot) comprising the area under positive peaks with the area under negative peaks subtracted or added;
- a length of any portion of the curve of the ECAP (e.g., the length of the curve from P1 to N2, L_P1toN2)
- any time defining the duration of at least a portion of the ECAP (e.g., the time from P1 to N2, t_P1toN2);
- a time delay from stimulation to issuance of the ECAP, which is indicative of the neural conduction speed of the ECAP, which can be different in different types of neural tissues;
- a rate of variation of any of the previous features, e.g., a difference between the previous value of the feature and the new value of the feature in the new stimulation period;
- any mathematical combination or function of these variables (e.g., H_N1 / FWHM_N1 would generally specify a quality factor of peak N1);
- any simplified version of the previous features that acts as a proxy for the specified feature. For example, instead of area under the curve, the sum of the absolute value of the sensed samples over the specified time interval; or instead of computing the length of the curve using Euclidean distance in a time interval, the length of the curve is computed as the sum of the absolute value of the difference of consecutive sensed samples; or instead of the height of N1 to P2 (H_PtoP), the maximum minus the minimum in a specified time interval, also known in statistics as the range of the sensed samples in a specified time interval. Such simplified features can be extracted directly using the hardware in the IPG;
- any of the previous features computed over any time interval t1 and t2, where t1 is the start of the time interval and t2 is the end of the time interval, and where t1 and t1 can be referred to the beginning of the stimulation pulse.
The feature extraction algorithm 140 can also determine one or more stimulation artifact features (SAFx), as described further below.

Once the feature extraction algorithm 140 determines one or more of these features, it may then adjust the stimulation that the IPG 100 provides, for example by providing new data to the stimulation circuitry 28 via bus 118. This is explained further in U.S. Patent Application Publications 2017/0296823 and 2019/0099602, which uses ECAP features to adjust stimulation. In one simple example, the feature extraction algorithm 140 can review the height of the ECAP (e.g., its peak-to-peak voltage) or the height of the ESG signal in any predefined time interval such as 0.6 ms to 2.2 ms, and in closed loop fashion adjust the amplitude I of the stimulation current to try and maintain the height in the interval or the height of the ECAP to a desired value.

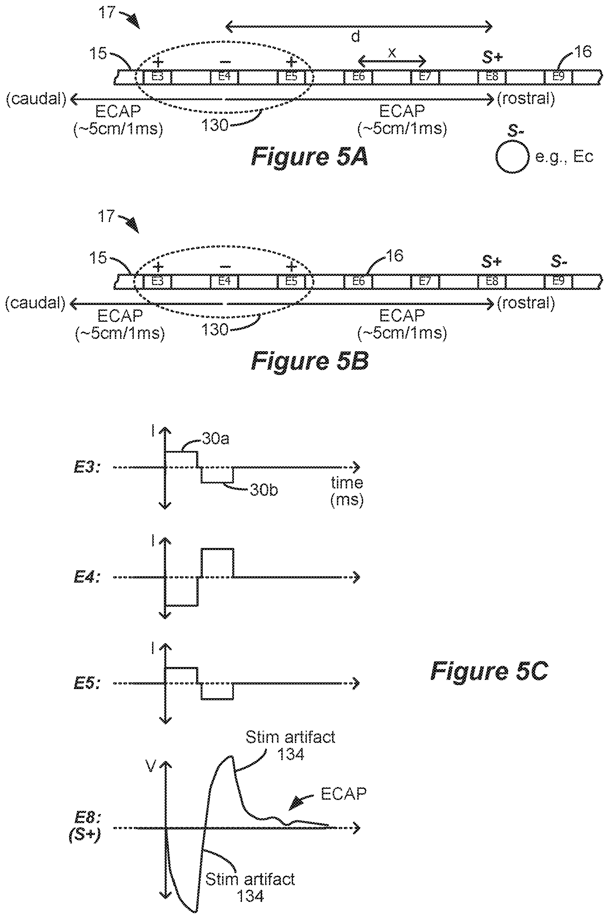

Figures 5A and 5B show an electrode array comprising a percutaneous lead 15, and show an example in which electrodes E3, E4 and E5 are used to produce pulses in a tripolar mode of stimulation, with (during the first phase 30a) E3 and E5 comprising anodes and E4 a cathode. Other electrode arrangements (e.g., bipoles, etc.) could be used as well. Such stimulation produces an electric field 130 in a volume of the patient's tissue around the selected electrodes. Some of the neural fibers within the electric field 130 will be recruited and fire, particularly those proximate to the cathodic electrode E4. It is expected that the sum of the neural fibers firing will mask signals indicative of pain in projection neurons which gate control theory suggests is the basis for SCS applications, thus providing the desired therapy relief. The recruited neural fibers in sum produce an ECAP, which can travel both rostrally toward the brain and caudally away from the brain. The ECAP passes through the spinal cord by neural conduction with a speed which is dependent on the neural fibers involved in the conduction. In one example, the ECAP may move at a speed of about 5 cm / 1 ms.

The ESG signal including the ECAP is preferably sensed differentially using two electrodes, and Figures 5A and 5B show different examples. In Figure 5A, a single electrode E8 on the lead 15 is used for sensing (S+), with another signal being used as a reference (S-). The sensing electrode S+ is spaced from the stimulation (or the stimulating electrodes) by a distance, d. In this example, the sensing reference S- comprises a more distant electrode in the electrode array 17 or (as shown) the case electrode Ec. In Figure 5B, two lead-based electrodes are used for sensing, with such electrodes either being adjacent or at least relatively close to one another. Specifically, in this example, electrode E8 is again used for sensing (S+), with adjacent electrode E9 providing the reference (S-). This could also be flipped, with E8 providing the reference (S-) for sensing at electrode E9 (S+). Sensing a given ECAP at different electrodes can allow the feature extraction algorithm 140 to determine the time difference between the arrival of the ECAP at each of the electrodes. If the distance x between the electrodes is known, the feature extraction algorithm 140 can then compute speed of the ECAP. As noted above, ECAP speed is indicative of the neural fibers involved in neural recruitment and conduction, which can be interesting to know in its own right, and which may comprise a feature used to adjust the stimulation provided by the stimulation circuitry 28. Sensing reference S- could also comprise a fixed voltage provided by the IPG 100, such as ground, in which case sensing would be said to be single-ended instead of differential.

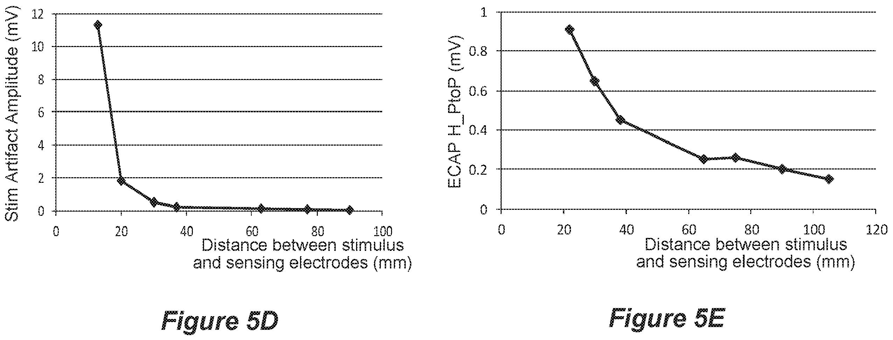

Figure 5C shows waveforms for the stimulation program, as well as the signal that would appear in the tissue at sensing electrode E8 (S+). As well as including the ECAP to be sensed, the ESG signal at the sensing electrode S+ also includes a stimulation artifact 134. The stimulation artifact 134 comprises a voltage that is formed in the tissue as a result of the stimulation, i.e., as result of the electric field 130 applied. As described in U.S. Patent Application Publication 2019/0299006, the PDACs and NDACs used to form the currents in the tissue have high output impedances. This can cause the voltage in the tissue to vary between ground and the compliance voltage VH used to power the DACs, which as noted earlier can be a high voltage (e.g., as high as 18V). The magnitude of the stimulation artifact 134 at a given sensing electrode S+ or its reference S- can therefore be high (e.g., tens to hundreds of miliVolts), and significantly higher than the magnitude of the ECAP. The magnitude of the stimulation artifact 134 at the sensing electrodes S+ and S- is dependent on many factors. For example, the stimulation artifact 134 will be larger if the stimulation-to-sense distance d is smaller, as shown in Figure 5D. The stimulation artifact 134 is also generally larger during the provision of the pulses, although it may still be present even after the pulse (i.e., the last phase 30b of the pulse) has ceased due to the capacitive nature of the tissue, which keeps the electric field 130 from dissipating immediately.

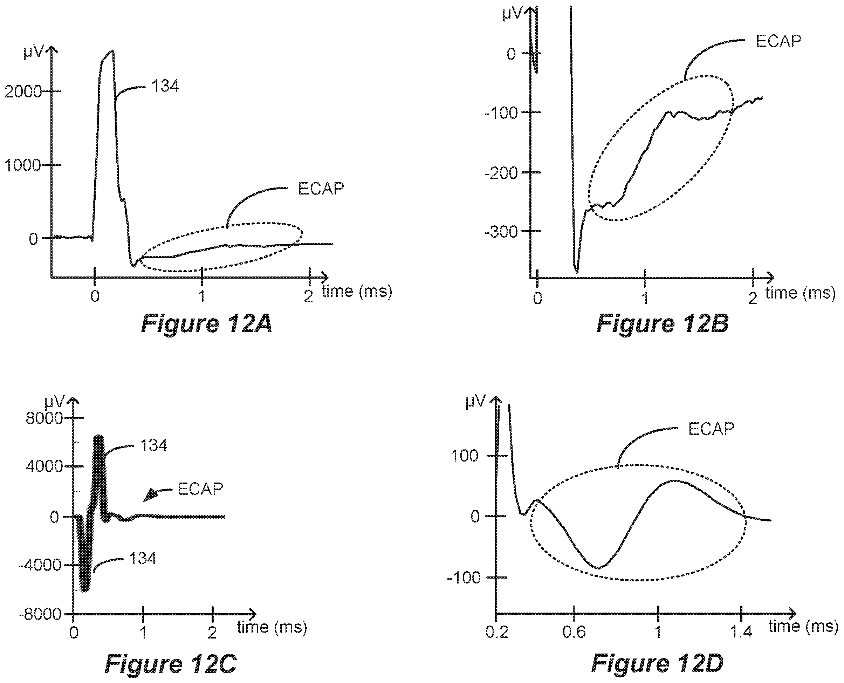

Realize that the ESG signal as shown at the sensing electrode S+ in Figure 5C is idealized. Figures 12A-12D show actual recorded ESG traces. Figure 12A shows an ESG trace taken in a human subject, in which a monophasic pulse is used for stimulation, and is followed by passive charge recovery. The stimulation artifact 134 corresponding to the pulse is highlighted, as in the ECAP. Figure 12B magnifies a portion of Figure 12A so that the ECAP can be better seen. Figure 12C shows an ESG trace taken on a human subject for a biphasic pulse stimulus. Again, the stimulation artifact 134 and ECAP are shown. Figure 12D magnifies a portion of Figure 12C so that the ECAP can be better seen.

Conventional wisdom teaches that the stimulation artifact 134 is an impediment to sensing, and thus techniques have labored to mitigate the effect of these artifacts at the sensing electrodes. This is because the relatively large-signal background stimulation artifact 134 can make resolution and sensing of the small-signal ECAP difficult at the sense amp circuit 110. The art thus teaches various ways to ameliorate the effects of stimulation artifacts from ESG signals in SCS systems. For example, the art teaches that it can be beneficial to increase the stimulation-to-sense distance d, because the stimulation artifact 134 would be smaller at a distant sensing electrode, and because the ECAP would pass a distant sensing electrode at a later time when the stimulation artifact 134 might have dissipated. See, e.g., U.S. Patent Application Serial No. 16/661,549, filed October 23, 2019. However, using a distant sensing electrode is not always possible or practical. For one, the electrode array 17 may simply not be large enough, and therefore no electrode may be suitably far enough away from the stimulating electrodes to ideally operate as the sensing electrode. Likewise, the magnitude of the ECAP also diminishes as distance from the stimulating electrodes increases due to neural response dispersion as it travels, as shown in Figure 5E, but at a much lower reduction rate compared to the artifact 134..

Differential sensing is another means of mitigating stimulation artifacts, because differential sensing can subtract the stimulation artifact 134 present at the sensing and reference electrodes S+ and S- to some degree as a common mode voltage, thus making the ECAP at the sensing electrode S+ easier to sense. Other techniques to mitigate the effect of stimulation artifact 134 beyond differential sensing have also been proposed. For example, SCS systems with sensing capability can include "blanking" capability, in which the input to the sense amp circuitry 110 is opened to prevent the stimulation artifact 134 from reaching the sense amp, at least in part. See, e.g., U.S. Patent Application Publications 2019/0299006 and 2019/0366094, and U.S. Patent Application Serial Nos. 16/821,602 and 16/821,617, both filed March 17, 2020.

Despite such conventional wisdom that teaches to mitigate or ameliorate the effects of stimulation artifacts in an ESG signal in an SCS system, the present inventors have recognized that stimulation artifacts in and of themselves can include useful information relevant to operation of the SCS implant and/or the status of the patient. In particular, stimulation artifact features as sensed can be used to determine a posture or activity of the patient, or more generally to adjust the stimulation program that the IPG is providing, as described further below. Furthermore, sensing of stimulation artifact features can be as useful as, and possibly even more useful than, information gleaned from sensing features of neural responses such as ECAPs.

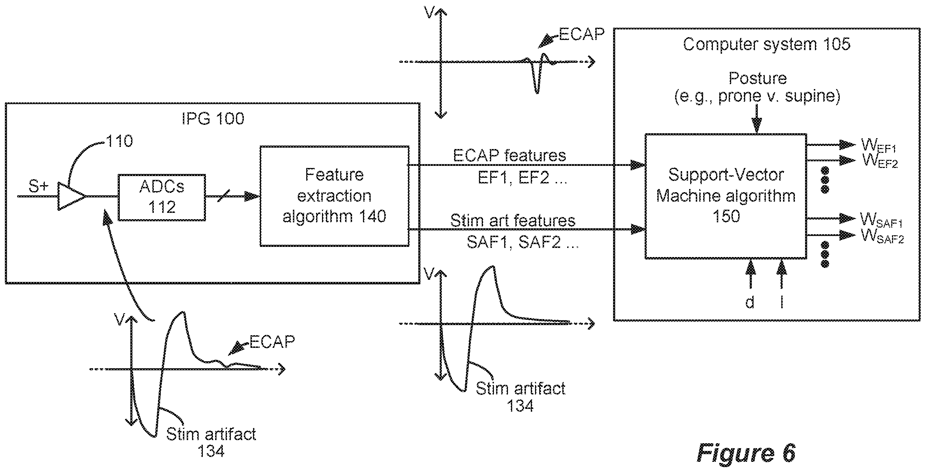

Figure 6 shows a system that was used to discover the relevance of stimulation artifacts in SCS sensing. The system includes the IPG 100 as previous described including the feature extraction algorithm 140. The IPG 100 was provided in an animal subject (a pig). Stimulation was provided having a first phase 30a (see, e.g., Fig. 2A) with a pulse width of 0.1 ms, and the amplitude I of the stimulation was varied. Charge recovery was provided, and thus the pulses provided were essentially biphasic in nature. However, pseudo-monophasic pulses could have been used as well, where pseudo-monophasic pulses consist of a monophasic pulse followed by a passive charge recovery. Either active or passive charge recovery could have been employed as described earlier. Sensing occurred at a sensing electrode S+, and the distance d of the sensing electrode relative to the stimulation was also varied. The signal as sensed at sensing electrode S+ for various combinations of d and I included both the stimulation artifact 134 and the ECAP, as shown in Figure 6. In this example, sensing was single-ended, not differential, with the sense amp circuitry 110 being referenced to a constant potential (Vamp, Fig. 4A) rather than to a reference electrode S- in contact with the tissue. Single-ended sensing was done for the specific purpose of sensing the stimulation artifact 134 rather than attempting to subtract it out from the measurement as differential sensing would tend to do. However, because differential sensing will not perfectly subtract out the stimulation artifact, differential sensing could have been used as well.

The feature extraction algorithm 140 was used to analyze the sensed signal, and was programmed to separate aspects of the sensed signal resulting from the stimulation artifact 134 and ECAP neural response. Such separation is relatively straight forward given the characteristic shapes of the stimulation artifact 134 and the ECAP. Note that different channels (electrodes) could be used to sense the stimulation artifact 134 and the ECAP. This alternative may be useful because it allows for the gain of the sense amps 110 to be adjusted. When sensing a smaller-signal ECAP at a first channel, the gain of the sense amp in that channel can be increased. When sensing the larger-signal stimulation artifact 134 at a second channel, the gain of the sense amp in that channel can be decreased. Still alternatively, the same electrode can be used to sense the ESG signal at different times (e.g., after different stimulation pulses), with the gain of the amplifier being increased at certain times to focus on ECAP sensing, and decreased at other times to focus on stimulation artifact sensing.

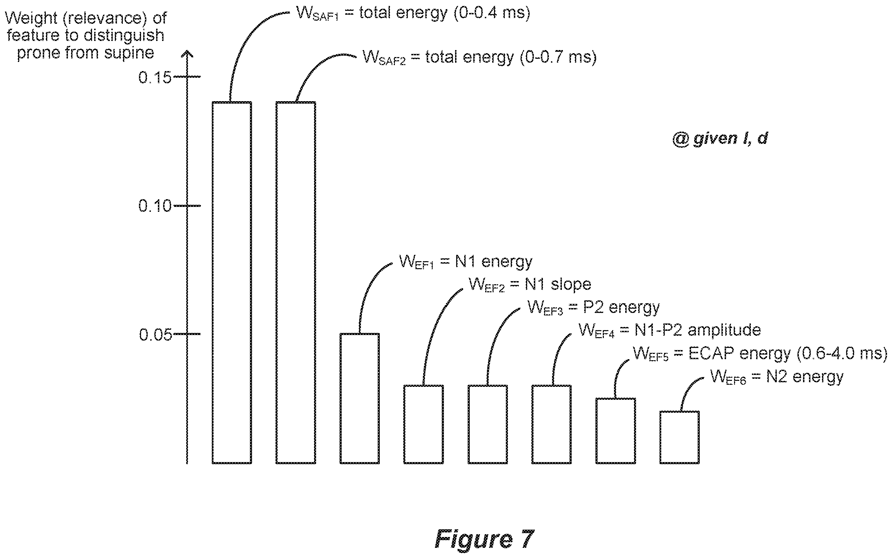

Once separated, the feature extraction algorithm 140 then determined a number of different features for the sensed stimulation artifacts (SAF1, SAF2, etc.), and for the sensed ECAPs (EF1, EF2, etc.). The ECAP features EFx could be those described earlier, such as peak-to-peak height, total energy (as determined by the area under portions of the ECAP curve), etc. The determined stimulation artifact features SAFx can be similar, and again can generally reflect the size and shape of the stimulation artifacts in different ways. Specific examples of ECAP features EFx and stimulation artifact features SAFx determined and evaluated during testing are described further with reference to Figure 7.

The ECAP features EFx and stimulation artifact features SAFx were transmitted to an external computer system 105 for analysis, and in particular to correlate the features to particular postures of the pig when the measurements were taken. In this regard, the pig subject was provided stimulation in both prone and supine positions, and features EFx and SAFx determined for different pulse amplitudes I and different stimulation-to-sense distances d. It would be expected that at least some of these features might change with posture, as posture can affect the distance of the electrode array 17 to the spinal cord. For example, changes in posture can cause the spinal cord to move within the CSF (cerebrospinal fluid) that surrounds the cord and is contained within the dura layer. The spinal cord is immersed in the CSF (cerebrospinal fluid) that cushions the spinal cord as it moves with body movement, respiration, heart beats, and activity such as laughing, talking, coughing, exercising, etc.). Body movement can cause the spinal cord and/or the electrode array 17 to move longitudinally, transversely, dorso-ventrally, or in any direction in the spinal column, or can cause the spinal cord and the array to become closer to each other, or farther from each other. See, e.g., USP 9,446,243. Such positional changes of the spinal cord and in the electrode array 17 will cause the tissue intervening between the stimulation and the sensing electrode to change, and it is therefore reasonable to anticipate that at least some of features EFx and SAFx detected at the sensing electrode S+ may change with changes in the distance between the spinal cord and the electrode array. If so, such features may be used to tell if there are changes in posture or activity state of the patient.

The computer system 105 included a support-vector machine algorithm 150 to analyze the features EFx and SAFx, and in particular to determine how significantly each of these features could distinguish between the two posture positions tested. Support-vector machine algorithm 150 represents a type of machine learning algorithm, and such algorithms are well known in the art. Note that the sensed waveform at sensing electrode S+ could also have been sent to the computer system 105 for analysis, and in this regard EFx and SAFx feature extraction (140) could also have taken place in the computer system. The output of the algorithm 150 in this example is weight W for each of the features (e.g., weight W_{EF1} for feature EF1), with each weight indicating the significance of the feature in discriminating between the tested postures.

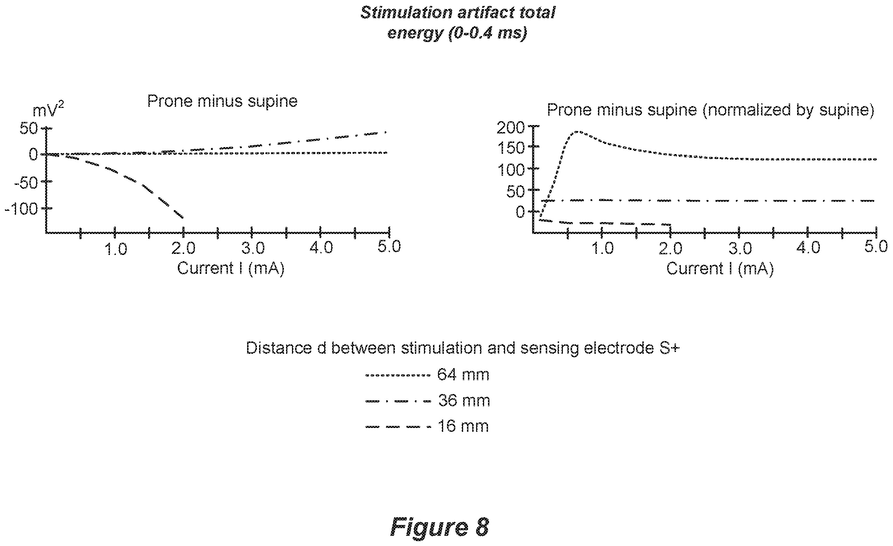

Figure 7 shows a bar chart of the weights of several of the features, with the most significant weights having the highest values. Such data is shown for a particular stimulation-to-sense distance d (36 mm) and a particular amplitude I of the stimulation current. As will be described later with reference to Figure 8, the features in question can change as a function of these variables.

As Figure 7 shows, certain features of the stimulation artifact 134 were noticed to be most significant in distinguishing between the prone and supine postures tested. In particular, the total energy of the stimulation artifact was noticed to be particularly significant. In this regard, Figure 7 shows two measures of total stimulation artifact energy, as measured over different time periods: from 0 to 0.4 ms (W_{SAF1}, when the stimulation artifact is most pronounced) and from 0 to 0.7 ms (W_{SAF2}, for the whole stimulation artifact until it becomes insignificant). Weights of lesser significance correspond to various ECAP features including the energy under the ECAP's N1 peak (W_{EF1}), the slope of N1 (W_{EF2}), the energy under the ECAP's P2 peak (W_{EF3}), the ECAP's peak-to-peak (N1-P2) amplitude (W_{EF4}), the energy under the ECAP in total (W_{EF5}, from 0.6 ms to 4.0 ms when the ECAP is present at the sensing electrode), and the energy under the ECAP's N2 peak (W_{EF6}). The various energy features were determined by squaring the voltages of the relevant curves or peaks and determining the resulting area underneath them. Other features could have been determined and evaluated for significance as well, and it is hypothesized in particular that other stimulation artifact features (e.g., amplitude) could also comprise features able to distinguish posture.

In any event, while the SCS art has focused significantly on analysis of ECAP features, and in so doing has labored to mitigate or remove the effect of stimulation artifacts from such measurements, Figure 7 shows that stimulation artifacts may carry significant information useful in an SCS system, and that such measurements may in fact be more useful than ECAPs in certain respects, such as patient posture determinations, including determinations for stimulation therapies above or below human perception threshold also known as sensory threshold.

Figure 8 shows further testing results, and in particular analyzes a particular stimulation artifact feature noticed in Figure 7 to have significance-total energy of the stimulation artifact from 0 to 0.4 ms. This feature as measured for both of the prone and supine posture states is compared in different graphs, and in different manners. The graph on the left shows the prone data minus the supine data, while the graph on the right shows the prone minus supine data normalized by the supine data. In either case, it can be seen that the prone and supine data differ, thus indicating the utility of this stimulation artifact feature to distinguish between these two posture states. Further, and as alluded to above, the graphs of Figure 8 shows how this stimulation artifact feature varies between the two posture states for different stimulation currents I and different stimulation-to-sense distances d. As such, these variables I and d are also relevant parameters that can affect the assessment in Figure 7. That is, different values for I and d may cause the weights as determined in Figure 7 to have different values. To assess if the effect of changes in the amplitude I on this feature for the different postures was the same, the right graph of Figure 8 that demonstrates the normalized difference of this feature is almost constant for a fixed distance d between stimulation and sensing. In other words, the actual difference of this feature between postures changes with amplitude I, but the normalized difference is constant because the changes in amplitude I affects both postures equivalently. In any event, Figures 7 and 8 show the utility of stimulation artifact features in an SCS system.

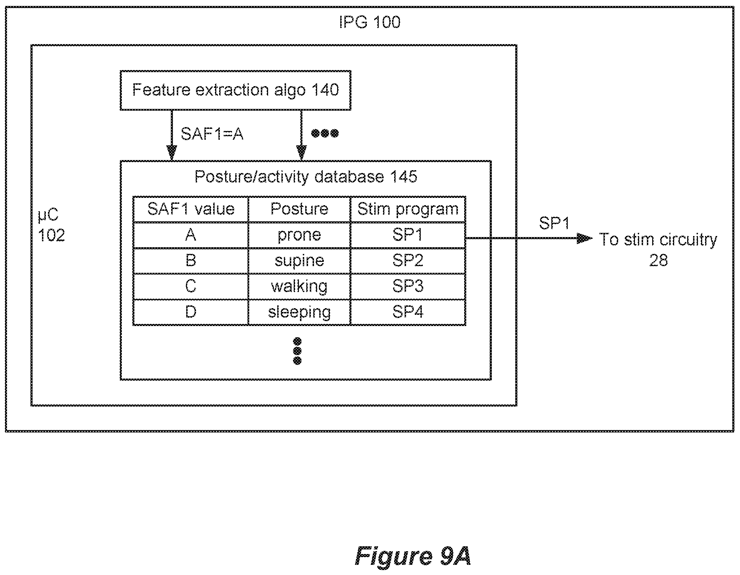

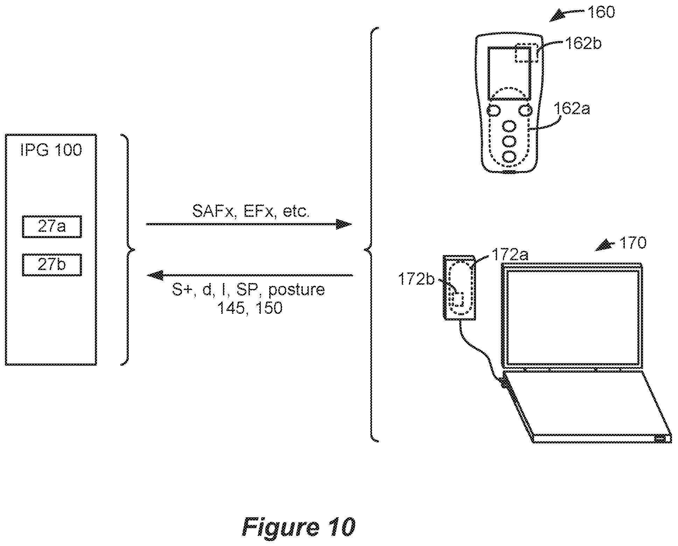

Figure 9A shows use of one or more stimulation artifact features SAFx in an IPG 100 to determine posture or activity, and to adjust stimulation therapy accordingly. In this example, the controller circuitry 102 of the IPG is programmed with a posture/activity database 145. This database 145 is preferably determined for each patient during a fitting session, as described later with reference to Figure 10. As shown, the database 145 correlates particular postures (such as prone or supine) or activities (such as walking or sleeping) with particular values of a stimulation artifact feature SAF1, which may again comprise the total stimulation artifact energy over a relevant time period (which time period may depend on the time period of the stimulating pulse). As can be seen, a prone posture correlates to a particular value A for feature SAF1 in the database. It should be understood that value A in database 145 can refer to a single value, or to a range of values. Although not shown, these various postures and activities can also be correlated in the database 145 with other stimulation artifact feature values (e.g., SAF2) and/or with other ECAP features EFx as well to the extent such features correlate significantly with posture or activity. However, for ease of illustration, use of just a single stimulation artifact feature SAF1 is shown.

Once the database 145 is populated for the patient, the IPG 100 can periodically sense the signal at the sensing electrode S+. Such sensing may occur periodically after a therapeutic pulse prescribed for the patient, or after a test pulse provided specifically for the purpose of sensing. Such sensing is preferably single ended, but may also be differential, because differential sensing can still allow the stimulation artifact to be sensed to some degree. Further, as sensing the stimulation artifact is a goal, blanking to keep the stimulation artifact from reaching the sense amp circuitry 110 is preferably disabled. In other words, blanking is preferably not used.

The sensed signal is passed to the feature extraction algorithm 140 described earlier, which can separate the stimulation artifact from the ECAP, and as necessary can determine features of each. In the example shown, the feature extraction algorithm has determined that a particular stimulation artifact feature SAF1 (e.g., total energy) has a value of A. This value can then be passed to the posture/activity database 145 to correlate it to a particular posture. In the example shown, a value of A for SAF1 correlates with a prone posture. The database 145 in this example further correlates each position or activity with a particular stimulation program SPx appropriate for that patient when in the posture or engaged in the activity. Each stimulation program preferably includes stimulation parameters, which may include stimulation amplitude (I), pulse width (PW), frequency (F), the active electrodes (E), the polarity of such active electrodes (P, whether anode or cathode), a percentage of current each active electrode is to receive (X%), and possibly still other parameters. If SAF1 equals A (or falls within the range of A), then the posture/activity database 145 will provide stimulation program SP1 to the stimulation circuity 28 (Fig. 4A) to adjust the patient's therapy accordingly. If SAF1 equals B, then stimulation program SP2 is provided, etc.

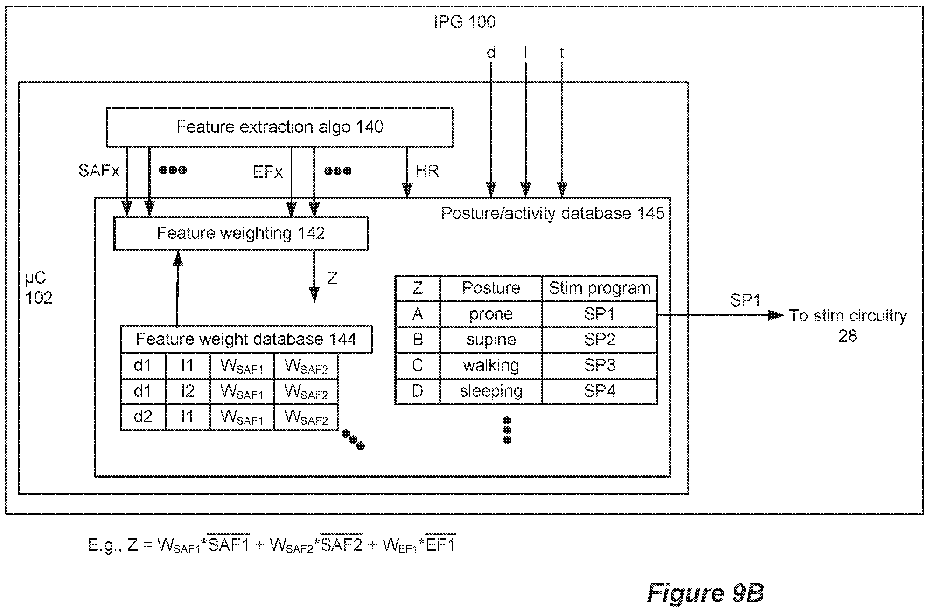

Figure 9B shows modification to feature extraction algorithm 140 and database 145, and shows additional variables that can be assessed when determining posture or activity, and when adjusting the patient's stimulation. In this example, a plurality of stimulation artifact features SAFx significant to differentiating patient posture are determined by the feature extraction algorithm 140, as are ECAP features EFx that may also have significance. These can be used to determine weighted factor Z indicative of posture or activity. In one example, the weights as determined in Figure 7 can be used. For example, the feature extraction algorithm can determine stimulation artifact features SAF1 (total energy from 0-0.4 ms) and SAF2 (total energy from 0- 0.7 ms), and even though it is less significant, may determine ECAP feature EF1 (N1 energy) as well. These features can be weighted in feature weighting logic 142 to determine the weighted factor Z. Because the different feature measurements (SAF1, SAF2, EF1) may have different values, it may be prudent to normalize them (S̅A̅F̅1̅, S̅A̅F̅2̅, E̅F̅1̅) to bring the magnitude of the values into relative parity. Each may then be multiplied by their weights W to determine the weighted factor Z as shown in the equation at the bottom of Figure 9B. The value of the weighted factor Z may then be used, at least in part, to determine the posture/activity, and hence the stimulation adjustment that is warranted.

The feature extraction algorithm 140 may also be used to determine the patient's heart rate, HR, which may also be used as a factor in determining posture/activity and stimulation adjustment. Technique for extracting heart rate in an SCS system from a sensed signal are disclosed in U.S. Patent Application Publication 2019/0290900, with which the reader is assumed familiar. Heart rate can be useful in addition to feature analysis to determining posture or activity. For example, a sleeping patient would have a lower heart rate, while a walking patient would have a higher heart rate.

Other factors may be useful for the posture/activity database 145 to consider when determining posture/activity and adjusting stimulation. For example, the time of day t can be consulted as well. This is particularly useful as a patient may tend to predictably change posture or activity at certain times of day. For example, the patient may work out from 7am to 8am; sit during working hours; walk during the lunch hour; sleep during evening hours, etc. Thus, time of day, like heart rate, can be useful to consider in addition to feature analysis when determining how patient stimulation therapy might be adjusted.

The amplitude of the stimulation current I and stimulation-to-sense distance d may also be useful to consider. As explained earlier with reference to Figure 8, significant factors such as stimulation artifact total energy can vary as a function of these variables. Thus, certain features may be more or less relevant at different values of I and d. For example, different values of I and d may change the weights W for the features, and thus I and d may be relevant for feature weighting logic 142 to consider. This is illustrated in Figure 9B using feature weight database 144, which provides logic 142 the relevant weights for different values of I and d. Of course, other parameters beyond I and d may be relevant and used as well, and I and d are simply noted as examples that may affect assessment of the measured stimulation artifact or ECAP features.

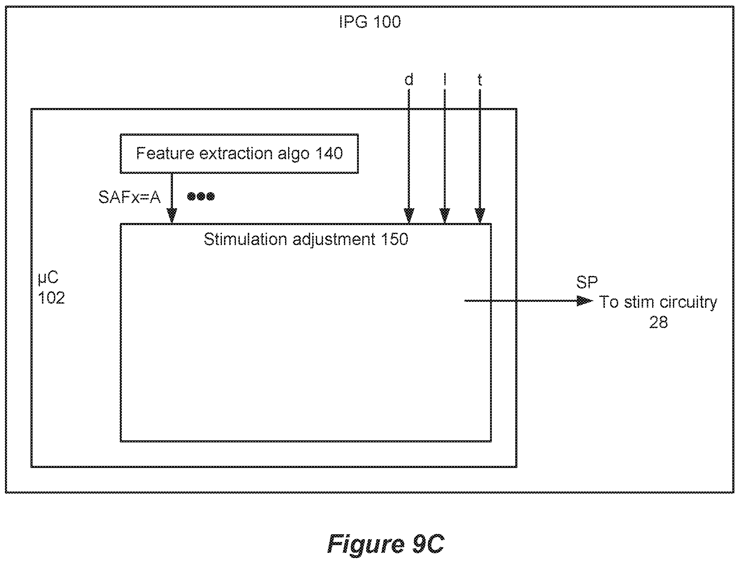

Figure 9C shows a more generic example where stimulation adjustment is made at least on the basis of one stimulation artifact feature. In this example, the measured feature(s) are provided to stimulation adjustment logic 150, possibly along with other relevant variables (such as ECAP features, amplitude I, distance d, time t, etc.). Stimulation adjustment logic 150 uses the measured at least one stimulation artifact feature (and possibly other variables) to adjust one or more stimulation parameters in a stimulation program (SP), such as amplitude, pulse width, frequency, active electrodes and their polarities and relative current percentages as discussed above. Stimulation adjustment as provided by stimulation adjustment logic 150 can occur in different manners. In one example, if the stimulation adjustment logic 150 notes that the stimulation artifact feature(s) have changed from baseline values, it may adjust one or more of the stimulation parameters in an attempt to keep the stimulation artifact feature(s) constant at such baseline values in a closed loop fashion. In one example, the stimulation adjustment logic 150 may randomly adjust one or more of the stimulation parameters until the feature(s) are brought back to baseline values. Further development and experimentation, or machine learning techniques, may also inform operation of stimulation adjustment logic 150. Although not shown in Figure 9C, stimulation adjustment logic 150 may also be assisted by a database like 145 (Figs. 9A and 9B) to make appropriate stimulation adjustments, even if such database doesn't store information relevant to patient posture or activity. In short, assessment of stimulation artifact features SAFx may be used to adjust stimulation parameters or programs even without a determination of posture or activity.

Figure 10 shows a system for "fitting" a patient, and in particular for programming the patient's IPG 100 with appropriate information-such as that contained in database 145 (Figs. 9A and 9B) or logic 150 (Fig. 9C) as useful to determining posture/activity or adjusting stimulation. In this regard, one skilled will understand that each patient's biology and symptoms are different, as is the position of the electrode array 17 in each patient's spinal column. It is thus beneficial to test each patient to determine appropriate baseline values for the sensed features, and in particular at different patient postures and activities.

The system of Figure 10 includes the IPG 100 as well as an external device in communication with the IPG 100. Such external device may comprise a patient hand-held external controller 160 or a clinician programmer 170. Details of such external devices are discussed in one example in U.S. Patent Application Publication 2019/0175915, and either can be used during a fitting procedure, although fitting usually occurs using the more-sophisticated clinician programmer typically found in a clinician's office or operating room. Communication between the external device and the IPG 100 can occur wirelessly, and the external controller can include a coil near-field magnetic induction antenna 162a or an RF antenna 162b respectively capable of communicating with the IPG's antennas 27a or 27b. Similarly, the clinician programmer 170 can include a coil near-field magnetic induction antenna 172a or an RF antenna 172b, which may be present in a communication wand 160 connected to the clinician programmer.

During a fitting procedure, the patient having IPG 100 may be instructed to position themselves in different postures or engage in different activities, such as those mentioned above. During such posture or activity, an appropriate stimulation program may be chosen for the patient that is satisfactory to improve or alleviate their symptoms (e.g., pain). As noted earlier, use of different stimulation programs may be warranted as different postures or activities can affect the positioning of the electrode array 17 in the spinal column. Similarly, one or more sensing electrodes S+ may be chosen for the patient, which may depend on the stimulation that is provided. As mentioned above, selection of the sensing electrode(s) S+ may depend on the electrodes that are used for stimulation, such that the distance d between sensing electrode(s) and the stimulation is neither too near to or too far. Choosing appropriate sensing electrode(s) may require verification, and thus the IPG 100 may transmit detected stimulation artifact features (SAFx) or ECAP features (EFx) as determined by the feature extraction algorithm 140 to the external device involved in fitting. This will allow the external user to verify that signals sensed at the sensing electrode(s) chosen are adequate to resolve the stimulation artifact and/or the ECAP artifacts. More specifically, the external user can verify that the sense amp circuitry 110 (Fig. 4A) is acceptably sensing such signals and that the feature extraction algorithm 140 is able to appropriately differentiate the stimulation artifact and ECAP portions of the sensed signal. In another example, the IPG 100 may transmit the entire digitized signal detected at the sensing electrode(s) to the external device, which external device may include the algorithm 140 and thus may be able to determine relevant features without the assistance of the IPG 100.

Suitable stimulation programs and sensing electrode(s) chosen for each posture/activity are then transmitted to the IPG 100. Stimulation is then provided with the patient in each posture or activity, with sensing occurring at the sensing electrode (S+). Such sensed features are then provided to the external device. (Again, feature extraction could also occur at the external device). This allows the external device to determine baseline measurements for the features at different positions/activities, in particular the stimulation artifact feature(s) SAFx. This in turn allows the external device to determine necessary information for database 145 or logic 150 and program them into the IPG 100 as Figure 10 shows. In particular, the external device can determine values or ranges for the relevant features (e.g., A, B, C, D) necessary to distinguish the postures or activity of the patient, and in turn stimulation program adjustment that may be warranted. In short, through the fitting procedure, the IPG 100 can be programmed to use at least one or more stimulation artifact features to determine patient posture or activity, and/or to determine how stimulation should be adjusted based on later detected stimulation artifact feature(s).

As well as adjusting stimulation, the IPG 100 can log relevant information and transmit it to an external device for patient or clinician review. Significantly, because the IPG 100 can determine patient posture or activity using at least the sensed stimulation artifact features, such logged information can be useful to review patient posture and activity changes over time, as well as the effectiveness of stimulation therapy provided to the patient during such postures and activities. This is particularly useful with respect to certain patient activities. For example, review of logged data can be relevant to assessing the quality of a patient's sleep, how much they are exercising, etc.

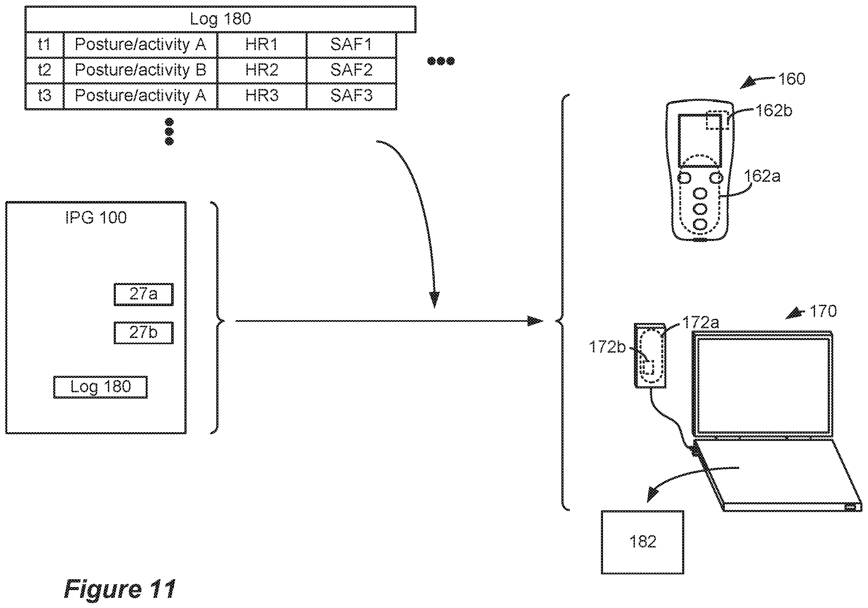

Accordingly, Figure 11 shows a data log 180 as stored in the IPG 100, and as may be transmitted from time to time to relevant external device 160 or 170 or to the Internet via an external device with internet connectivity. Information in the data log 180 can vary, but in one example may include a log of patient posture and/or activities at different times of day. In other words, the data in data log 180 can be used to determine daily patient activity maps for the patient. For example, the data log 180 in Figure 11 shows that the patient is involved in posture/activity A at time t1, which may be a time range; posture/activity B at time t2; posture/activity A at time t3, etc. The data log 180 may also include other relevant information, such as the heart rate HR detected during these times. Although not shown, the log 180 may include other variables as well, including the stimulation-to-sense distance d and the current amplitude I. These variables may be valuable to log and transmit as they may be changeable by the patient; in particular, the patient may use his external controller 160 to change the current amplitude I from time to time.

The data log 180 may further include information regarding the stimulation artifact features (SAFx) as determined at relevant times. This also may be useful to review, and further may be relevant to possibly re-adjusting the database 145 or logic 150 in the IPG 100. In any event, Figure 11 shows that detection of stimulation artifact features can be useful in its own right, even if such features are not used to adjust the stimulation that the IPG 100 provides.

The data in data log 180 can be used to analyze patient sleep quality, and to generate a sleep quality report 182. Such as report 182 would preferably be generated in the clinician programmer 170 as shown in Figure 11, but could also be generated in external devices having access to log 180 as well, such as the patent's external controller. Nocturnal body movements as reflected by posture or activity can be used as marker of sleep quality, and may also inform as to the patient's sleep stage, and the duration of those stages, which may be reflected in report 182. Report 182 may also include distribution of sleep positions, and reflect how often a patient's sleep position changes. Report 182 may include a statistic analysis of relevant data, and as such may include metric like average body movement per hours, and metrics relevant to how much time a patient was resting during the night or how much time a patient was resting per day. This data can span the days over months or years. In short, the report 182 generated from the data in log 180 can effectively comprise a sleep or rest tracker.
Further prefrred embodiments are described by the following items:
1. A method for operating a stimulator device, the stimulator device comprising a plurality of electrodes configured to contact a patient's tissue, the method comprising:
   providing stimulation at at least two of the electrodes;
   sensing a stimulation artifact over time at a sensing electrode comprising one of the electrodes different from the at least two electrodes that provide the stimulation, wherein the stimulation artifact comprises a signal formed by an electric field induced in the tissue by the stimulation;
   determining at least one feature of the stimulation artifact; and
   using at least the determined at least one stimulation artifact feature to determine a posture or activity of the patient.
2. The method of item 1, wherein the electric field is configured to recruit neural fibers in the tissue causing a neural response.
3. The method of item 2, further comprising:
   sensing the neural response at the sensing electrode;
   determining at least one feature of the neural response; and
   using the determined at least one neural response feature to determine the posture or activity of the patient.
4. The method of item 2, wherein the sensed stimulation artifact excludes the neural response.
5. The method of item 2, wherein sensing the stimulation artifact comprises subtracting the neural response from the stimulation artifact.
6. The method of item 2, wherein sensing the stimulation artifact occurs before arrival of the neural response at the sensing electrode.
7. The method of item 1, wherein the stimulator device is programmed with a database associating values or ranges of values of the at least one stimulation artifact with different of the postures or activities.
8. The method of item 7, wherein determining at least one feature of the stimulation artifact comprises determining a value for the stimulation artifact feature, and wherein using the determined at least one stimulation artifact feature to determine a posture or activity of the patient comprises using the determined stimulation artifact feature value to select from the database one of the postures or activities that is associated with a value or range of values that matches the determined stimulation artifact feature value.
9. The method of any of items 1-8, wherein the stimulation artifact is sensed at a sense amplifier in the stimulation device, and wherein the stimulation artifact is sensed at the sense amplifier in a single-ended manner using a fixed reference potential as a reference.
10. The method of any of items 1-8, wherein the stimulation artifact is sensed at a sense amplifier in the stimulation device, and wherein the stimulation artifact is sensed at the sense amplifier differentially using another one of the electrodes as a reference.
11. The method of items 9 or 10, wherein the stimulation artifact is not blanked at the sense amplifier.
12. The method of any of items 1-11, wherein the at least one stimulation artifact feature is indicative of an energy of the stimulation artifact.
13. The method of any of items 1-12, wherein the at least two electrodes are spaced at a distance from the sensing electrode, and further comprising using the distance to determine the posture or activity of the patient.
14. The method of any of items 1-13, wherein the stimulation has an amplitude, and further comprising using the amplitude to determine the posture or activity of the patient.
15. The method of any of items 1-14, further comprising transmitting the determined posture or activity of the patient to an external device.
16. A method for operating a stimulator device, the stimulator device comprising a plurality of electrodes configured to contact a patient's tissue, the method comprising:
   providing stimulation at at least two of the electrodes, wherein the stimulation induces an electric field in the tissue, wherein the electric field is configured to recruit neural fibers in the tissue causing a neural response;
   sensing a signal over time at a sensing electrode comprising one of the electrodes different from the at least two electrodes that provide the stimulation, wherein the signal comprises a stimulation artifact formed by the electric field and the neural response; and
   processing the sensed signal to determine at least one feature of the stimulation artifact and to determine at least one feature of the neural response.
17. The method of item 16, wherein the sensed signal is digitized prior to processing the sensed signal.
18. The method of items 16 or 17, wherein the signal is sensed at a sense amplifier in the stimulation device, and wherein the signal is sensed at the sense amplifier in a single-ended manner using a fixed reference potential as a reference.
19. The method of items 16 or 17, wherein the signal is sensed at a sense amplifier in the stimulation device, and wherein the signal is sensed at the sense amplifier differentially using another one of the electrodes as a reference.
20. The method of items 18 or 19, wherein the signal is not blanked at the sense amplifier.
21. The method of any of items 16-20, wherein the at least one stimulation artifact feature is indicative of an energy of the stimulation artifact.
22. The method of any of items 16-21, wherein the stimulator device comprises a Spinal Cord Stimulator device.
23. The method of any of items 16-22, further comprising using one or more of the determined at least one stimulation artifact feature and the determined at least one neural response feature to adjust the stimulation.
24. The method of item 23, wherein the method is repeated to continually adjust the stimulation.
25. The method of item 23, wherein the stimulator device is programmed with a database associating different stimulation programs with values or ranges of values of the at least one stimulation artifact feature or with values or ranges of the at least one neural response feature.
26. The method of any of items 16-25, further comprising using one or more of the determined at least one stimulation artifact feature and the determined at least one neural response feature to determine a posture or activity of the patient.
27. The method of item 26, wherein the stimulator device is programmed with a database associating different of the postures or activities with values or ranges of values of the at least one stimulation artifact or with values or ranges of the at least one neural response feature.
28. The method of item 26, further comprising transmitting the determined posture or activity of the patient to an external device.
29. The method of item 26, further comprising storing a log of the determined posture or activity of the patient as a function of time in the stimulator device.
30. The method of item 29, further comprising transmitting the log to an external device.
31. A method for operating a stimulator device, the stimulator device comprising a plurality of electrodes configured to contact a patient's tissue, the method comprising:
   providing stimulation at at least two of the electrodes;
   sensing a stimulation artifact over time at a sensing electrode comprising one of the electrodes different from the at least two electrodes that provide the stimulation, wherein the stimulation artifact comprises a signal formed by an electric field induced in the tissue by the stimulation;
   determining at least one feature of the stimulation artifact; and
   using at least the determined at least one stimulation artifact feature to adjust the stimulation.
32. The method of item 31, wherein the electric field is configured to recruit neural fibers in the tissue causing a neural response.
33. The method of item 32, further comprising:
   sensing the neural response at the sensing electrode;
   determining at least one feature of the neural response; and
   using the determined at least one neural response feature to adjust the stimulation.
34. The method of item 32, wherein the sensed stimulation artifact excludes the neural response.
35. The method of item 32, wherein sensing the stimulation artifact comprises subtracting the neural response from the stimulation artifact.
36. The method of item 32, wherein sensing the stimulation artifact occurs before arrival of the neural response at the sensing electrode.
37. The method of any of items 31-36, further comprising using the determined at least one stimulation artifact feature to determine a posture or activity of the patient.
38. The method of item 37, wherein the stimulator device is programmed with a database associating values or ranges of values of the at least one stimulation artifact with different of the postures or activities.
39. The method of item 38, wherein determining at least one feature of the stimulation artifact comprises determining a value for the stimulation artifact feature, and wherein using the determined at least one stimulation artifact feature to determine a posture or activity of the patient comprises using the determined stimulation artifact feature value to select from the database one of the postures or activities that is associated with a value or range of values that matches the determined stimulation artifact feature value.
40. The method of any of items 31-39, wherein the stimulation artifact is sensed at a sense amplifier in the stimulation device, and wherein the stimulation artifact is sensed at the sense amplifier in a single-ended manner using a fixed reference potential as a reference.
41. The method of any of items 31-39, wherein the stimulation artifact is sensed at a sense amplifier in the stimulation device, and wherein the stimulation artifact is sensed at the sense amplifier differentially using another one of the electrodes as a reference.
42. The method of items 40 or 41, wherein the stimulation artifact is not blanked at the sense amplifier.
43. The method of any of items 31-42, wherein the at least one stimulation artifact feature is indicative of an energy of the stimulation artifact.
44. The method of any of items 31-43, wherein the at least two electrodes are spaced at a distance from the sensing electrode, and further comprising using the distance to adjust the stimulation.
45. The method of any of items 31-44, wherein the stimulation has an amplitude, and further comprising using the amplitude to adjust the stimulation.

## Claims

1. A stimulator device, comprising:
a plurality of electrodes configured to contact a patient's tissue;
stimulation circuitry configured to provide stimulation at at least two of the electrodes, wherein the stimulation induces an electric field in the tissue, wherein the electric field is configured to recruit neural fibers in the tissue causing a neural response;
a sense amplifier configured to sense a signal over time at a sensing electrode comprising one of the electrodes different from the at least two electrodes that provide the stimulation, wherein the signal comprises a stimulation artifact formed by the electric field and the neural response; and
control circuitry configured to process the sensed signal to determine at least one feature of the stimulation artifact and to determine at least one feature of the neural response.

2. The stimulator device of claim 1, further comprising analog-to-digital converter circuitry configured to digitize the sensed signal prior to processing the sensed signal at the control circuitry.

3. The stimulator device of claims 1 or 2, wherein the signal is sensed at the sense amplifier in a single-ended manner using a fixed reference potential as a reference.

4. The stimulator device of claims 1 or 2, wherein the signal is sensed at the sense amplifier differentially using another one of the electrodes as a reference.

5. The stimulator device of claims 3 or 4, wherein the signal is not blanked at the sense amplifier.

6. The stimulator device of any of claims 1-5, wherein the at least one stimulation artifact feature is indicative of an energy of the stimulation artifact.

7. The stimulator device of any of claims 1-6, wherein the stimulator device comprises a Spinal Cord Stimulator device.

8. The stimulator device of any of claims 1-7, wherein the control circuitry is further configured to use one or more of the determined at least one stimulation artifact feature and the determined at least one neural response feature to adjust the stimulation.

9. The stimulator device of claim 8, wherein the control circuitry is configured to continually adjust the stimulation.

10. The stimulator device of claim 8, wherein the control circuitry is programmed with a database associating different stimulation programs with values or ranges of values of the at least one stimulation artifact feature or with values or ranges of the at least one neural response feature.

11. The stimulator device of any of claims 1-10, wherein the control circuitry is further configured to use one or more of the determined at least one stimulation artifact feature and the determined at least one neural response feature to determine a posture or activity of the patient.

12. The stimulator device of claim 11, wherein the control circuitry is programmed with a database associating different of the postures or activities with values or ranges of values of the at least one stimulation artifact or with values or ranges of the at least one neural response feature.

13. The stimulator device of claim 11, wherein the control circuitry is further configured to store a log of the determined posture or activity of the patient as a function of time in the stimulator device.

14. The stimulator device of claim 13, wherein the control circuitry is further configured to transmit the log to an external device.

15. A non-transitory computer readable medium comprising instructions that when executed by control circuitry are configured to operate a stimulator device, the stimulator device comprising a plurality of electrodes configured to contact a patient's tissue, wherein the instructions are configured to:
provide stimulation at at least two of the electrodes, wherein the stimulation induces an electric field in the tissue, wherein the electric field is configured to recruit neural fibers in the tissue causing a neural response;
sense a signal over time at a sensing electrode comprising one of the electrodes different from the at least two electrodes that provide the stimulation, wherein the signal comprises a stimulation artifact formed by the electric field and the neural response; and
process the sensed signal to determine at least one feature of the stimulation artifact and to determine at least one feature of the neural response.
